## Europäisches Patentamt

## European Patent Office

## Office européen des brevets

(11) Publication number: **0 121 255**
**B1**

## (12) EUROPEAN PATENT SPECIFICATION

(45) Date of publication of patent specification: **21.09.88**

(51) Int. Cl.⁴: **A 61 M 16/01**

(21) Application number: **84103555.3**

(22) Date of filing: **30.03.84**

(54) **Closed circuit anaesthesia device.**

(30) Priority: **05.04.83 NL 8301191**

(43) Date of publication of application:
**10.10.84 Bulletin 84/41**

(45) Publication of the grant of the patent:
**21.09.88 Bulletin 88/38**

(84) Designated Contracting States:
**AT BE CH DE FR GB IT LI SE**

(56) References cited:
**GB-A- 889 164**
**US-A-3 378 005**
**US-A-4 182 599**
**US-A-4 188 946**

(73) Proprietor: **Hellige GmbH**
**Postfach 728 Heinrich-von-Stephan-Strasse 4**
**D-7800 Freiburg im Breisgau (DE)**

(72) Inventor: **Schepp, Ronald, Dr.**
**Milliadeplein 15**
**NL-2651 GP Berkel en Rodenrijs (NL)**

(74) Representative: **Patentanwälte TER MEER -**
**MÜLLER - STEINMEISTER**
**Mauerkircherstrasse 45**
**D-8000 München 80 (DE)**

Courier Press, Leamington Spa, England.

## Description

The invention relates to an anaesthesia device with breathing return, comprising a casing and a separation between a primary side, which is provided with a connection to a respiration control machine, and a secondary side, which is provided with a connection to the patient and divided in two branches one of which is provided with a carbonic acid absorber, control means for detecting the composition and the volume of the respiratory air, a volume-displacement detector, the information output of which is connected to said control means, and connections for the supply of the required gases to the secondary side.

For administering narcosis a so-called semi-open system presents two extremely important disadvantages. In the first place a particularly great amount of gases is used, which is very expensive. In the second place the great surplus of gases enters into the operating room. The so-called semi-closed systems to obviate these objections in part, but nevertheless the amounts of gas used are quite considerable. Closed circuit anesthesia is also being applied, but only by a small number of anesthetists.

In order to give some idea it can be stated that with semi-closed systems in practice 2 to 3 liters of oxygen per minute are supplied and 4 to 6 liters of laughing gas. The real consumption by the patient is in the order of 1/4 liter of oxygen per minute and, initially, 1.5 to 2 liters of laughing gas, within a few minutes decreasing to a few hundred milliliters per minute. Anesthetists for reasons of medical security with the semi-closed systems feel compelled to apply such great surplus amounts; even when taking amounts of 2 liters of oxygen and 4 liters of laughing gas per minute, this implies cost of the gas of about Hfl. 24.- per hour.

The adverse health effects don't exist for patients who are exposed once, during several hours at maximum, to the effect of narcotics. The problem is with the operating room personnel, who breath the gases from the narcotizing apparatus entering the atmosphere in the operating room during the greater part of their full working time. Clear indications exist that this causes diseases, so that both in the Netherlands and in other countries legislation is being prepared for the protection of personnel in operating rooms. One of the possible solutions for the problem is the provision of an exhaust installation, the costs of which per group of operating rooms in a hospital are in the order of Hfl. 250.000.-.

The advantages of a fully closed anesthesia system have been recognized long since. In the first place the amount of gases required is reduced to what is taken up directly by the patient. This means that the above-mentioned cost of Hfl. 24.- per hour can be reduced to Hfl. 2.- per hour, for oxygen and narcotic gas. The problem of pollution of the atmosphere in the operating room is as a matter of course non-existent with a fully closed system. Apart from these two external factors, which are very striking, there is a great series of advantages from the point of view of the patient and the medical treatment, respectively. Some of these are: in a closed system, the narcotic gases stay warmer and are more humid, cold and not-humid gases in a non-closed system entailing a considerably greater risk of problems with the lungs. With a closed system, the oxygen consumption can be determined. The amount of narcotic taken up can be determined accurately, which allows a better control of the depth of anaesthesia. Still further advantages from the medical point of view can be found in the anaesthesiological literature.

A semi-open respiratory system is described in US—A—4 182 599. This system makes use of a cylinder with two chambers. A rolling diaphragm is secured to the inner wall of the cylinder and separates the two chambers. The exhaled gas mixture is discharged through an outlet passage and is vented to atmosphere through a check valve. GB—A—889 164 describes a respirator with closed gas system comprising an anaesthesia device as specified in the prior art portion of Claim 1. This respirator is provided with a conventional gas analysing apparatus and with a certain number of different types of valves. The composition of the respiratory gas mixture circulating in the system is indicated by a pointer. Lack of, for instance, laughing gas or nitrogen, indicated by the pointer, can be compensated by manually attaching a source thereof.

Despite the manifest advantages, closed systems are still applied by a small number of anaesthetists yet, of which those who consider the advantages to prevail over the disadvantages are faced with unavoidable further disadvantages up to now. These disadvantages can be summarized concisely as the necessity of an additional assistant for the continuous manual control of the amounts of gas and the manual steering of respiration, the risk of errors in the amounts administered, particularly by a faulty reaction to phenomena occurring unexpectedly with the patient under treatment.

The invention starts from a system connected to a machine for controlling respiration, for shortness called ventilator. With the known semi-open systems and the semi-closed systems modulating is done with a machine, but it can also be done by hand, whilst up to now with closed systems modulating is done exclusively by hand. The separation between the primary side of the device, connected to the respiration control machine which thus takes care of rhythmically pressurizing the secondary side and this secondary side, which is connected to the patient's respiratory system, comprises a balloon included in a box. The interior of the balloon is connected to the secondary side. When modulating is done manually, it is done by squeezing the balloon by hand.

The object of the present invention is to provide a device of the fully closed type, capable of functioning fully automatically, and this, as a matter of course, with complete security. In this way, the closed system becomes applicable at large scale, so that the advantages of this system can be gained everywhere.

The anaesthesia device according to the present invention is characterized in that the separation between the primary and the secondary sides comprises a cylindrical piston sealingly movable with respect to a cylindrical housing wall by means of a rolling seal which simultaneously serves as a reservoir for the respiratory gas and as said volume-displacement detector, the direction of circulation of the respiration gas mixture through said two branches of the secondary side is ensured by a blower, the information output of an oxygen sensor is connected with said control means for communication with an oxygen supply in such a manner that there is a continuous supply of the amount of oxygen required as an average, and in that at the end of every expiration phase the volume lost as compared with the previous stroke is determined and the information output communicates, via said control means with the supplies for the gases in such a manner that the lost volume detected is immediately replenished in the form of a narcotic gas.

With the invention, it becomes possible to continuously determine how much of the gases have been used by the patient, and thereby it becomes possible also to adjust this use continuously. This idea can be realized in practice with a greater accuracy and reliability than is the case with a closed anaesthesia circuit having manual operation.

More particularly, for the purpose of separating the primary and the secondary sides it is possible to use a device which has been available under the term "dry spirometer" long since. Such dry spirometers have an extremely low inertia, and, therefore, a very fast response. When this apparatus is used, it is possible to obtain, already from the first inhaling stroke after the beginning of administering the narcotic gas, information about the anaesthetic process, which is useful for the virtually perfect control of the process. For a good understanding it is of importance to note that the anaesthetic effect of narcotic gas is dependent on the partial pressure which this gas reaches in the arterial blood. The rate at which this happens is determined, apart from the alveolar ventilation, by the difference in concentration between the interior of the lungs and the blood flow. At the beginning of the narcosis, therefore, the narcotic gas is taken up at a fast rate; 2 to 3 liters of laughing gas per minute as the case may be. After 5 or 6 minutes, the narcotic gas content in the blood has reached 90% already of the maximum value attainable. The take-in which is initially very high, has then decreased already to the order of 250 ml per minute and will go down still a little further to a more or less constant value which is required as a compensation for the evaporation of the narcotic gas occurring continuously at the surface of the body. From the course of the take-in of the narcotic gas, the anaesthetist will be able to draw particularly interesting conclusions about the patient, upon which he could react immediately to control the whole anaesthetic process. Up to now, it was a common habit, at the start, to "wash the patient's blood in", with several liters of a narcotic gas, and thereupon close the system. But then the opportunity has passed already to obtain the most important information. With the system according to the invention, which is completely closed as from the start, all important information from the first respiration stroke after the start of administering narcotic gas can be recorded, processed and presented in a useful manner to the anesthetist. Resuming, it means that with the device according to the invention, the anesthetist has a better control of the manner in which the patient reacts on the narcotic gases administered.

The device according to the invention provided in known manner with an oxygen sensor, is preferably realized in this way that the information output of the oxygen sensor is connected with the control unit for communication with the oxygen supply in such manner that there is a continuous supply of the amount of oxygen required as an average, and that at the end of every expiration phase the volume lost as compared with the previous stroke is determined and the information output communicates, via the control unit, with the supplies for the gases in such manner that the lost volume detected is immediately replenished in form of narcotic gas.

Apart from this, the invention offers possibilities for measuring a considerable number of parameters which are of great medical importance.

A special aspect is the risk of the arising of leaks in the system. With the closed system, as it has hitherto been applied, with manual respiration control, accidents could be caused, as a result of unobserved leaks in the system, because of a reduction in the oxygen concentration. With the system according to the invention this cannot happen anymore. In case of leakage the ratio between oxygen and narcotic gas remains constant. In case of changing the connections for narcotic gas and oxygen, the valve for the narcotic will automatically close and within a short period the system will contain oxygen only.

The device according to the invention also has features which make it fit to be used as an auxiliary respiration apparatus, particularly for intensive care. In this case the information outputs of the volume-displacement detector are connected with the oxygen supply in such manner that the volume lost with every stroke is entirely compensated for in form of oxygen.

By the addition of a device capable of building up pressure on the primary side, the device may also function as an independent respiration control apparatus.

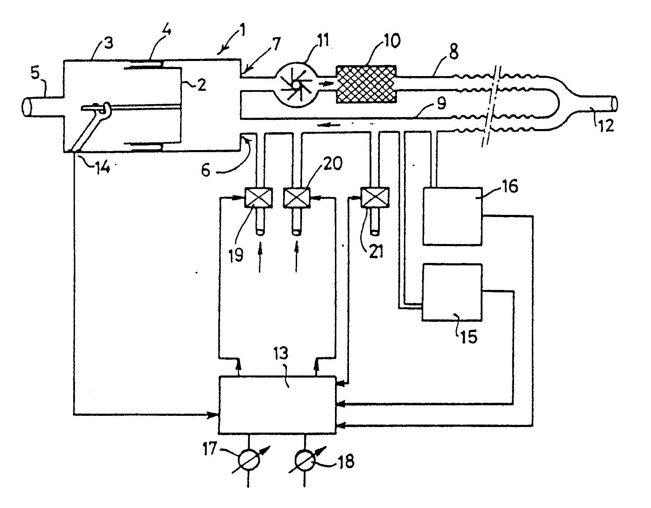

The invention will now be clarified with reference to the accompanying drawing.

The single figure shows a diagram of the device.

The kernel of the device is the volume-displacement detector, schematically represented here as a dry spirometer 1, in which a light bell 2 is arranged within a cylindrical casing 3, while placing a rolling seal 4 in between.

The bell and the rolling seal together constitute a complete separation between the primary space, at the left hand side of the figure, which has a connection 5, and the secondary space, at the right hand side, having two connections 6 and 7 respectively. With connection 5 the spirometer can be connected to the respiration control device which may be entirely of known type and which produces volume-displacements.

The connections 6 and 7 communicate with two branches 8, 9 of the circuit. In the one branch 8 there is a $CO_2$-absorber 10 and a blower 11, which ensures that there can only be circulation in the sense indicated. The two branches 8 and 9 meet again at 12, where the connection to the patient can be made. The control unit 13 is provided with several information inputs. In the first place it receives data from the information output 14 of spirometer 1. Next in any case also detected are data from the oxygen analyzer, schematically indicated by 15 and eventually also the data from carbon dioxide analyzer 16. The two or three of them detect the gas in the circuit. Furthermore, as a matter of course, there is an opportunity to feed certain set-values to control unit 13. An information input 17 may serve for the set-value of the volume of the system and input 18 for the set-value of the oxygen concentration. Outputs from control unit 13 will at least comprise an output to a control valve 19 for the narcotic gas and an output to a control valve 20 for the oxygen.

Under command of the respiration control machine, via the oscillating spirometer bell 2, the respiration of the patient will be kept under control. On the secondary side the system, together with the contents of the lungs and the further respiratory system of the patient, is closed. All carbonic acid gas expired by the patient is absorbed. From the analysis of the composition of the gas it can be determined how much oxygen the patient consumes as an average, and this amount consumed is replenished continuously.

Subsequently, at the end of every expiration stroke of the patient, the lost volume is determined by means of spirometer 1, and this is replenished immediately by narcotic gas. Then the whole operation of the device is automatic, although the anesthetist, as a matter of course, does have the opportunity to change the set-values for the various parameters, depending on the anesthetic method which he wants to apply, the development of the process, particularly the reactions of the patient, etc.

Of course, all information taken can be processed and made visible in any desired manner, and also be recorded in view of noting the patient's history.

Apart from the essential features of the system, described above, there is a possibility to determine a great number of parameters which are of medical interest. A first example of these has been represented in the drawing, in form of a supply valve 21 for helium gas. This is a neutral gas, offering the possibility to determine the FRC (functional residual capacity) of the lungs. The course of consumption of the narcotic gas, for example laughing gas, and of the oxygen consumption provides information, e.g. about the respiration, the circulation, the heart-minute volume and, more generally, the patient's entire metabolic condition.

Also in the vicinity of connection 5 one may simply add a device for introducing, during a short time, an overpressure at the primary side of the system, for example in the order of 5 cm head of water (which device may be a tube submerged a 5 cm depth in an amount of water). The ratio between the change of volume $\Delta V$ as a result of this pressure difference $\Delta P$ is a measure for the lung compliance, and for $\Delta FRC$ in relation to $\Delta P$.

The system can be provided with an aperture to observe the effects of a very fast concentration change of the narcotic gas. As a matter of course other gas-analyzers can be connected with the system.

Resuming, the device according to the application permits a much better control of the patient, particularly on what his body does with the narcotic gases administered, and this better than with the closed circuit anesthesia as applied up to now. These advantages add to the most important advantages, as discussed in the preamble, of a saving in gas consumption which per operating room per annum, on the basis of a normal week's working time, lies at about Hfl. 40.000.- per annum, and furthermore, the prevention of pollution of the operating room atmosphere.

The device according to the application also has properties which make it fit to be employed just as an auxiliary device for respiration control, particularly for intensive care. In this case the information outputs of the volume-displacement detector are connected with the oxygen supply in such manner that the volume lost with every stroke is replenished entirely in form of oxygen.

By addition of a device capable of building up pressure at the primary side, the device is capable of functioning as an independent respiration control apparatus.

**Claims**

1. Anaesthesia device with breathing return, comprising
—a casing and a separation (1, 2) between a primary side, which is provided with a connection (5) to a respiration control machine, and a secondary side, which is provided with a connection (6, 7) to the patient and divided in two branches (8, 9) one of which is provided with a carbonic acid absorber (10),
—control means (13) for detecting the composition and the volume of the respiratory air,
—a volume-displacement detector, the information output (14) of which is connected to said control means, and

—connections (19, 20) for the supply of the required gases to the secondary side, characterized in that

—the separation between the primary and the secondary sides comprises a cylindrical piston (2) sealingly movable with respect to a cylindrical housing wall (3) by means of a rolling seal (4) which simultaneously serves as a reservoir for the respiratory gas and as said volume-displacement detector,

—the direction of circulation of the respiration gas mixture through said two branches (8, 9) of the secondary side is ensured by a blower (11),

—the information output of an oxygen sensor (15) is connected with said control means (13) for communication with an oxygen supply (20) in such a manner that there is a continuous supply of the amount of oxygen required as an average, and in that

—at the end of every expiration phase the volume lost as compared with the previous stroke is determined and the information output (14) communicates, via said control means (13) with the supplies for the gases in such a manner that the lost volume detected is immediately replenished in the form of a narcotic gas.

2. Device according to claim 1, characterized in that the secondary side is provided with a connection (21) for an analytic gas, such as helium, such that during the anaesthesia the functional residual capacity of the lungs of the patient can be determined.

3. Device according to claim 1, characterized in that at the primary side means are provided for temporarily increasing the pressure of the respiration control machine, such that from the change of volume resulting from this at the secondary side, the lung compliance can be determined.

4. Device according to claim 1, characterized in that means are provided to measure respiration air parameters, processing them, making them readable, and/or registering them such that furthermore, any of the following determinations can be made:

—the course of take in of narcotic,

—the course of take in of oxygen,

—determining the ratio between the change of the functional residual capacity and the change of pressure.

5. Device according to any of the preceding claims, characterized in that, for use as an auxiliary respiration control machine for intensive care, the information output of the volume-displacement detector communicates, via the control means (13), with the oxygen supply in such manner that the volume lost with every stroke is fully replenished in form of oxygen.

6. Respiration control apparatus, comprising a device according to any of claims 1 to 5, provided at the primary side thereof with a device for building up pressure.

**Patentansprüche**

1. Anästhesiegerät mit Atem-Rückführung, mit

—einem Gehäuse und einer Abtrennung (1, 2) zwischen einer Primärseite, die mit einer Verbindung (5) zu einem Beatmungssteuergerät versehen ist, und eine Sekundärseite, die eine Verbindung (6, 7) zu einem Patienten aufweist und in zwei Zweige (8, 9) aufgeteilt ist, von denen einer einen Kohlensäureabsorber (10) enthält,

—einer Steuervorrichtung (13) zur Messung der Zusammensetzung und des Volumens der Atemluft,

—einem Detektor für Volumenverschiebung, dessen Ausgang (14) mit der Steuervorrichtung verbunden ist, und mit

—Anschlüssen (19, 20) zur Zuführung der erforderlichen Gase zur Sekundärseite, dadurch gekennzeichnet, daß

—die Trennung zwischen der Primär- und der Sekundärseite einen zylindrischen Kolben (2) aufweist, der in Bezug auf die zylindrische Gehäusewand (3) bewegbar und mittels einer Rolldichtung (4) abgedichtet ist, die gleichzeitig als Reservoir für das geatmete Gas und als Detektor für eine Volumenverschiebung dient,

—die Richtung der Zirkulation des Atmungsgases durch die zwei Zweige (8, 9) der Sekundärseite mittels eines Gebläses (11) bestimmt ist,

—der Informationsausgang eines Sauerstoffsensors (15) mit der Steuervorrichtung (13) zur Beinflussung einer Sauerstoffzufuhr (20) so verbunden ist, daß eine kontinuierliche Zuführung der im Mittel erforderlichen Sauerstoffmenge sichergestellt ist, und daß

—am Ende jeder Ausatmungsphase das im Vergleich mit dem vorhergehenden Hub verlorene Volumen bestimmt wird und der Informationsausgang (14) die Zuführung der Gase über die Steuervorrichtung (13) in der Weise beeinflußt, daß der gemessene Volumenverlust sofort in Form von Narkosegas ergänzt wird.

2. Vorrichtung nach Anspruch 1, dadurch gekennzeichnet, daß die Sekundärseite mit einem Anschluß (21) für ein Analysegas, wie z.B. Helium versehen ist, so daß während der Anästhesie die funktionelle Restkapazität der Lungen des Patienten bestimmt werden kann.

3. Vorrichtung nach Anspruch 1, dadurch gekennzeichnet, daß die Primärseite Vorrichtungen zur zeitweisen Vergrößerung des Druckes des Beatmungssteuergeräts aufweist, so daß aus der daraus resultierenden Veränderung des Volumens an der Sekundärseite die Dehnbarkeit der Lunge bestimmt werden kann.

4. Vorrichtung nach Anspruch 1, dadurch gekennzeichnet, daß Vorrichtungen zur Messung der Parameter der Atmungsluft, sowie zu ihrer Verarbeitung, Darstellung, und/oder Registrierung vorgesehen sind, so daß außerdem die folgenden Größen bestimmt werden können:

—der Verlauf der Aufnahme von Narkotika,

—der Verlauf der Aufnahme von Sauerstoff,

—das Verhältnis zwischen der Änderung der funktionalen Restkapazität und der Änderung des Druckes.

5. Vorrichtung nach einem der vorhergehenden Ansprüche, dadurch gekennzeichnet, daß der Informationsausgang des Detektors für die Volumenverschiebung bei der Verwendung als Steuergerät für Hilfsbeatmung bei Intensivbehandlung über die Steuervorrichtung (13) die Sauerstoffzufuhr in der Weise beeinflußt, daß das bei jedem Hub verlorene Volumen vollständig in Form von Sauerstoff ergänzt wird.

6. Beatmungssteuergerät mit einer Vorrichtung nach einem der Ansprüche 1 bis 5, das an seiner Primärseite mit einer Vorrichtung zur Erzeugung eines Druckes ausgestattet ist.

**Revendications**

1. Appareil d'anesthésie avec retour respiratoire, comprenant

—une enveloppe et une séparation (1, 2) entre un côté primaire, qui est muni d'une connexion (5) à une machine de contrôle de la respiration, et un côté secondaire, qui est muni d'une connexion (6, 7) avec le malade et divisé en deux branches (8, 9) dont une est munie d'un absorbeur d'acide carbonique (10),

—un moyen de contrôle (13) pour détecter la composition et le volume de l'air respiratoire,

—un détecteur de déplacement de volume, dont la sortie d'information (14) est reliée au dit moyen de contrôle, et

—des connexions (19, 20) pour la fourniture des gaz nécessaires au côté secondaire, caractérisé en ce que

—la séparation entre les côtés primaire et secondaire comprend un piston cylindrique (2) pouvant se déplacer de façon étanche par rapport à une paroi de logement cylindrique (3) au moyen d'un joint coulissant (4) qui joue simultanément le rôle de réservoir pour le gaz respiratoire et le rôle dudit détecteur de déplacement de volume,

—le sens de circulation du mélange de gaz respiratoire à travers lesdites deux branches (8, 9) du côté secondaire est assuré par un ventilateur (11),

—la sortie d'information d'un détecteur d'oxygène (15) est reliée audit moyen de contrôle (13) aux fins de communication avec une alimentation en oxygène d'une manière telle qu'il y a un apport continu de la quantité d'oxygène requise en moyenne, et en ce que

—à la fin de chaque phase s'expiration on détermine le volume perdu par comparaison avec la course précédente et la sortie d'information (14) communique via ledit moyen de contrôle (13) avec les alimentations en gaz de manière telle que le volume perdu détecté est immédiatement compensé sous la forme d'un gaz narcotique.

2. Dispositif selon la revendication 1, caractérisé en ce que le côté secondaire est muni d'une connexion (21) pour un gaz analytique, comme l'hélium, de manière que pendant l'anesthésie on puisse déterminer la capacité résiduelle fonctionnelle des poumons du malade.

3. Dispositif selon la revendication 1, caractérisé en ce qu'au côté primaire des moyens sont prévus pour accroître temporairement la pression de la machine de contrôle de la respiration, de manière qu'à partir du changement de volume qui en résulte au côté secondaire, on puisse déterminer la réponse pulmonaire.

4. Dispositif selon la revendication 1, caractérisé en ce que des moyens sont prévus pour mesurer les paramètres aériens de la respiration, les traiter, les rendre lisibles, et/ou les enregistrer de manière qu'en outre on puisse faire l'une quelconque des déterminations suivantes:

—le trajet d'absorption de narcotique,

—le trajet d'absorption d'oxygène,

—détermination du rapport entre le changement de capacité fonctionnelle résiduelle et le changement de pression.

5. Dispositif selon l'une quelconque des revendications précédentes, caractérisé en ce que, en vue d'une application comme machine de contrôle auxiliaire de la respiration pour des soins intensifs, la sortie d'information du détecteur de déplacement de volume communique, via le moyen de contrôle (13), avec l'alimentation en oxygène de manière telle que le volume perdu avec chaque course soit pleinement compensé sous forme d'oxygène.

6. Appareil de contrôle de la respiration, comprenant un dispositif selon l'une quelconque des revendications 1 à 5, avec à son côté primaire un dispositif pour établir la pression.